Europäisches Patentamt

European Patent Office

Office européen des brevets

(11) Numéro de publication: **0 422 981 A1**

## (12) DEMANDE DE BREVET EUROPEEN

(21) Numéro de dépôt: **90402683.8**

(22) Date de dépôt: **28.09.90**

(51) Int. Cl.⁵: **G01N 33/68,** G01N 33/02,
C12N 5/12

Une requête en rectification de la première page a été présentée conformément à la règle 88 CBE. Il est statué sur cette requête au cours de la procédure engagée devant la division d'examen (Directives relatives à l'examen pratiqué à l'OEB, A-V, 2.2).

Le (Les) microorganisme(s) a (ont) été déposé(s) auprès Collection Nationale de Cultures de Microorganismes ( Institut Pasteur ) sous les numéros I 907, 908, 909.

(30) Priorité: **10.10.89 FR 8913181**

(43) Date de publication de la demande:
**17.04.91 Bulletin 91/16**

(84) Etats contractants désignés:
**AT BE CH DE DK ES FR GB GR IT LI LU NL SE**

(71) Demandeur: **INSTITUT NATIONAL DE LA RECHERCHE AGRONOMIQUE (INRA)**
**147, rue de l'Université**
**F-75341 Paris Cédéx 07(FR)**

(72) Inventeur: **Paraf, Alain**
**Maison Vert, Cellettes**
**F-41341 Les Montils(FR)**

(74) Mandataire: **Ores, Irène et al**
**CABINET ORES 6, Avenue de Messine**
**F-75008 Paris(FR)**

(54) **Réactifs et procédé de dosage immunochimique du degré de dénaturation de substances protéiques.**

㉗ Le réactif immunochimique d'identification et de quantification d'une substance protéique-test contenue dans un produit alimentaire est constitué par au moins un anticorps monoclonal dirigé contre une protéine-test contenue dans ledit produit alimentaire et dénaturée par un traitement thermique effectué à une température de traitement donnée, pendant une durée déterminée.

Application dudit réactif à l'identification et à la quantification d'une protéine-test présente dans un produit alimentaire à tester.

EP 0 422 981 A1

## REACTIFS ET PROCEDE DE DOSAGE IMMUNOCHIMIQUE DU DEGRE DE DENATURATION DE SUBSTAN-CES PROTEIQUES

La présente Invention est relative à des perfectionnements aux réactifs et aux procédés de dosage immunochimique du degré de dénaturation de substances protéoques, conformes au brevet principal.

Le Brevet principal n° 2 631 705 a pour but de prourvoir à des réactifs immunochimiques propres à permettre le dosage du degré de dénaturation de substances protéiques telles que celles contenues dans des produits alimentaires soumis à des traitements de dénaturation thermiques, physiques, chimiques ou enzymatiques. Conformément au Brevet principal, on met en oeuvre des anticorps pour identifier et quantifier un composant (principalement des composants protéiques et polysaccharidiques) présent dans un produit alimentaire quelconque et pour déterminer à quelle température le produit alimentaire a été chauffé - en particulier pour contrôler si le traitement de conservation auquel a été soumis le produit alimentaire est suffisant du point de vue technique et du point de vue hygiénique. Le Brevet principal a proposé d'utiliser des anticorps polyclonaux ou monoclonaux dirigés contre une protéine-test présente dans le produit alimentaire à contrôler, telle que l'ovalbumine, pour différencier par la technique ELISA, la présence d'ovalbumine native initialement présente dans le produit à contrôler, préalablement au traitement thermique et la présence d'ovalbumine dénaturée par chauffage, et de déterminer à quelle température le produit avait été chauffé (50°C, 65°C, 85°C, 100°C, 130°C, en particulier).

La présente Invention a pour but de pourvoir à des réactifs immunochimiques constitués par des anticorps non seulement aptes à déterminer à quelle température la protéine-test a été chauffée, mais également aptes à déterminer la durée pendant laquelle la protéine-test a été chauffée à la température déterminée. De plus la présente Invention a pour but de pourvoir à des anti-corps dirigés contre une protéine-test dénaturable par les températures de chauffage auxquelles sont soumis des produits alimentaires à conserver, qui permettent de déterminer les températures de chauffage avec une précision d'au moins 5°C, qui peut même atteindre 1°C.

La présente Invention a également pour but de pourvoir à un test, notamment à un test sandwich qui fait intervenir la méthode ELISA, pour identifier la durée du traitement thermique subi par les produits alimentaires à tester et la température du traitement thermique avec une précision d'au moins 5°C.

La présente Invention a pour objet un réactif immunochimique d'identification et de quantification d'une substance protéique-test contenue dans un produit alimentaire à controler, et de reconnaissance de la température et de la durée du traitement thermique auquel a été soumis ledit produit alimentaire à contrôler, caractérisé en ce qu'il est constitué par au moins un anticorps monoclonal dirigé contre une protéine-test contenue dans ledit produit alimentaire et dénaturée par un traitement thermique effectué à une température de traitement donnée, pendant une durée déterminée.

Selon un mode de réalisation avantageux du réactif immunochimique conforme à la présente invention, celui-ci est constitué par une batterie d'anticorps monoclonaux dont un anticorps est dirigé contre ladite protéine-test à l'état natif, et dont au moins un autre anticorps est dirigé contre ladite protéine-test dénaturée par un traitement thermique effectué à une température de traitement donnée, pendant une durée déterminée.

La présente Invention a en outre pour objet un kit d'identification et de quantification d'une protéine-test présente dans un produit alimentaire à tester, par la méthode ELISA, à l'aide d'anticorps dirigés contre la protéine native et contre au moins un état de dénaturation de ladite protéine par la chaleur, caractérisé en ce qu'il comprend une batterie d'anticorps monoclonaux dirigés contre divers états de ladite protéine-test, respectivement dirigés contre ladite protéine-test à l'état natif et dirigés contre des états de dénaturation de ladite protéine-test provoqués par des traitements thermiques effectués à différentes températures, éventuellement voisines les unes des autres, et contre des états de dénaturation de ladite protéine-test provoqués par lesdits traitements thermiques appliqués pendant différentes durées, lesquels anticorps monoclonaux sont aptes à réaliser l'immunocapture de l'antigène correspondant, à savoir de ladite protéine-test dans l'état correspondant aux anticorps dirigés contre elle, ledit kit étant en outre caractérisé en ce qu'il comprend des anticorps polyclonaux propres à permettre l'identification de l'antigène lié à l'anticorps monoclonal spécifique correspondant.

Outre les dispositions qui précèdent, l'invention comprend encore d'autres dispositions, qui ressortiront de la description qui va suivre.

L'invention sera mieux comprise à l'aide du complément de description qui va suivre, que se réfère à des exemples de mise en oeuvre d'un test de reconnaissance de la température et de la durée d'un traitement thermique auquel est soumise une substance protéique-test présente dans des conserves alimentaires, exemples illustrés par les dessins annexés, dans lesquels :

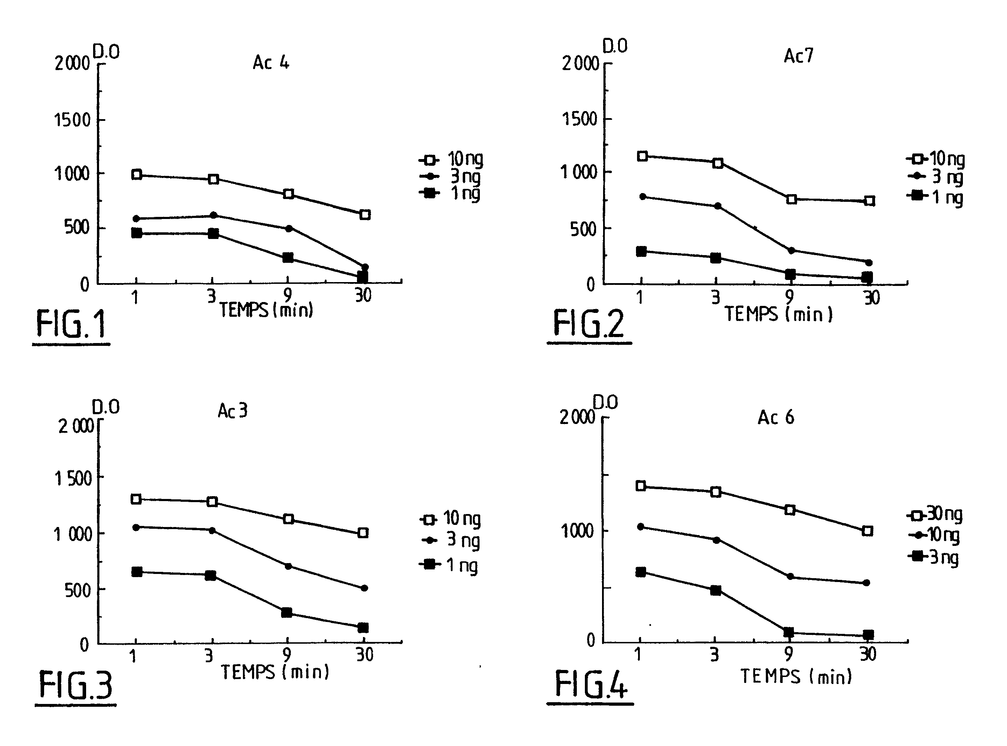

les figures 1 à 5 représentent la reconnaissance de diverses quantités d'une substance protéique-test traitée à une température déterminée pendant des durées variables, à l'aide d'anticorps monoclonaux dirigés contre cette substance.

Il doit être bien entendu, toutefois, que ces exemples et dessins et les parties descriptives correspondantes, sont donnés uniquement à titre d'illustration de l'objet de l'invention, dont ils ne constituent en aucune manière une limitation.

Exemple 1 Reconnaissance de la température à laquelle une substance protéique-test a été chauffée.

Plusieurs anticorps monoclonaux dirigés contre une protéine-test susceptible d'être contenue en tant qu'additif dans des conserves alimentaires ont été produits par la méthode de KOHLER et MILSTEIN : de l'ovalbumine native et de l'ovalbumine dénaturée par un traitement thermique à 75° C appliqué pendant 30 minutes, ont été respectivement injectées à des souris convenablement choisies qui ont ensuite été sacrifiéees et dont les cellules de rate ont été fusionnées avec des cellules de plasmocytome de souris.

Après sélection des hybridomes secrétant des anticorps dirigés respectivement contre l'ovalbumine native et contre l'ovalbumine dénaturée par un traitement thermique, on a isolé une pluralité d'anticorps monoclonaux qui ont été utilisés dans un test sandwich ELISA pour déterminer les températures de chauffage auxquelles avait été soumise de l'ovalbumine.

Il va de soi que bien que la protéine-test utilisée dans le cadre de la présente Invention soit de l'ovalbumine, l'invention s'applique néanmoins à n'importe quelle protéine-test dénaturable par un traitement thermique dans les gammes de température utilisées en conserverie, que cette protéine-test soit ajoutée au produit alimentaire conservé ou qu'elle y soit naturellement présente.

Les anticorps monoclonaux suivants ont été isolés et utilisés dans le test de détermination de la température du traitement thermique, conforme à la présente Invention.

| N° d'identification de l'anticorps | Dénomination de l'anticorps | Nature |
|---|---|---|
| 3 | MB4 D8 | IgG$_1$ |
| 4 | QA6 B5 | IgG$_1$ |
| 6 | KA4 B4 | IgG$_1$ |
| 7 | AD6 C4 | IgG$_1$ |
| 8 | BC4 F2 | IgM |
| 9 | IB4 E5 | IgG$_1$ |

Les hybridomes BC4F2, IB4E5, KA4B4, qui secrètent ces anticorps monoclonaux ont fait l'objet d'un dépôt auprès de la CNCM tenue par l'INSTITUT PASTEUR, sous les N° I-907, I-908 et I-909 en date du 10 Octobre 1989.

Le test de détermination de la température du traitement thermique mis en oeuvre est un test sandwich ELISA utilisant d'une part les anticorps monoclonaux précités pour l'immunocapture de l'antigène et d'autre part des anticorps polyclonaux de lapin NAC$_2$ pour identifier l'antigène capturé.

La protéine-test constituant l'antigène à identifier a été de l'ovalbumine chauffée pendant 10 minutes à différentes températures.

Dans le Tableau I ci-après, on montre les densités optiques obtenues en utilisant quatre des anticorps monoclonaux précités pour l'immunocapture. Ce tableau montre une nette différence entre 70° C et 75° C d'une part et 75° C et 80° C d'autre part, lorsque l'ovalbumine est présente dans le milieu à une concentration de 50 ng/ml.

TABLEAU I

| Reconnaissance de la présence de 50 ng/ml d'ovalbumine au bout de 10 minutes de traitement thermique, à l'aide d'anticorps monoclonaux, dans un test sandwich ELISA | | | | | | | | |
|---|---|---|---|---|---|---|---|---|
| Anticorps numéro | Non traitée | Densités optiques pour Traitée à | | | | | | |
| | | $50°C$ | $65°C$ | $70°C$ | $75°C$ | $80°C$ | $85°C$ | |
| 4 | 1700 | 1700 | 1600 | 1500 | 1400 | 200 | 0 | |
| 6 | 1000 | 950 | 950 | 850 | 850 | 200 | 0 | |
| 8 | 0 | 0 | 0 | 0 | 300 | 650 | 650 | |
| 9 | 0 | 0 | 0 | 0 | 400 | 800 | 800 | |

Exemple 2 Détermination de la durée du traitement thermique.

Le rôle de la durée de chauffage de l'ovalbumine a été examiné en réalisant un test sandwich ELISA dans lequel l'immunocapture a été réalisée à l'aide, respectivement, de cinq des anticorps monoclonaux mentionnés plus haut.

Une solution d'ovalbumine (10 µg/ml) a été chauffée pendant différentes périodes à 75°C et différentes dilutions ont été testées.

Les figures 1 à 4 annexées font apparaître les quantités d'antigène testé en fonction de l'anticorps monoclonal mis en oeuvre pour l'immunocapture, et les durées pendant lesquelles l'ovalbumine a été chauffée.

D'importantes différences ont été observées dans de telles conditions :
- pour une même concentration en antigène (3ng/ml) avec un traitement thermique similaire à 75°C :
   1) avec l'anticorps 4, 80 % de la D.O. (densité optique) sont perdus entre 3 et 30 minutes ;
   2) avec l'anticorps 7, 60 % de l'activité sont perdus entre 3 et 30 minutes ;
   3) avec l'anticorps 3, 40% de l'activité seulement sont perdus entre 3 et 30 minutes,et
   4) avec l'anticorps 9, 90 % de l'activité sont perdus entre 1 et 9 minutes.

Plus la concentration d'antigène est faible, plus le test est sensible, mais le moment auquel l'activité disparait est sensiblement le même.

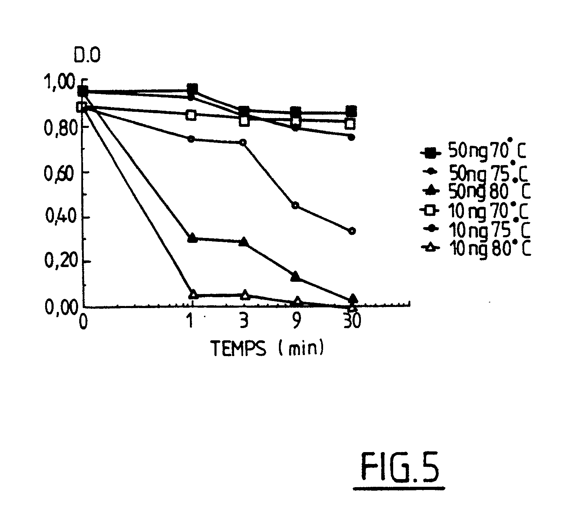

Ces résultats sont radicalement différents lorsque l'on compare les résultats obtenus respectivement pour un traitement thermique à 75°C et pour un traitement thermique à 80°C : la figure 5 annexée montre que pour 75°C, l'expression épitopique n'est pas sensiblement modifiée, que la concentration en ovalbumine soit de 50 ou de 10 ng/ml, alors qu'à une température de 80°C, 70 à 90% des épitopes disparaissent en une minute.

Il ressort des exemples qui précédent que si l'on utilise un anticorps monoclonal spécifique pour l'immunocapture, on peut utiliser un anticorps polyclonal spécifique de lapin ou de souris pour la détection de l'antigène capturé, que celui-ci soit de l'ovalbumine, ceux dans lesdits exemples, ou une autre protéine-test dénaturable aux températures de traitement mise en oeuvre en conserverie, que la protéine-test soit une protéine ajoutée au produit alimentaire conservé ou qu'elle soit normalement présente dans ce dernier.

Ces exemples montrent que les réactifs immunochimiques conformes à la présente Invention permettent :
   1) de détecter avec une précision de 5°C et même parfois d'1°C, la température à laquelle l'antigène a été chauffé ;
   2) d'identifier la durée pendant laquelle le traitement thermique a été appliqué.

Ainsi que cela ressort de ce qui précède, l'invention ne se limite nullement à ceux de ses modes de réalisation et d'application qui viennent d,être décrits de façon plus explicite ; elle en embrasse au contraire toutes les variantes qui peuvent venir à l'esprit du technicien en la matière, sans s'écarter du cadre, ni de la portée, de la présente invention.

**Revendications**

1° Réactif immunochimique d'identification et de quantification d'une substance protéique-test contenue dans un produit alimentaire à contrôler, et de reconnaissance de la température et de la durée du traitement thermique auquel a été soumis ledit produit alimentaire à contrôler, caractérisé en ce qu'il est constitué par au moins un anticorps monoclonal dirigé contre un état de dénaturation déterminé d'une protéine-test contenue dans ledit produit alimentaire et dénaturée par un traitement thermique effectué à une température de traitement donnée, pendant une durée déterminée.

2° Réactif immunochimique selon la revendication 1, caractérisé en ce qu'il est constitué par une batterie d'anticorps monoclonaux dont un anticorps est dirigé contre ladite protéine-test à l'état natif, et dont les autres anticorps sont dirigés contre différents états de dénaturation de ladite protéine-test dénaturée par un traitement thermique effectué à une température de traitement donnée, pendant une durée déterminée.

3° Kit d'identification et de quantification d,une protéine-test présente dans un produit alimentaire à'tester, par la méthode ELISA, à l'aide d'anticorps dirigés contre la protéine native et contre au moins un état de dénaturation de ladite protéine par la chaleur, caractérisé en ce qu'il comprend une batterie d'anticorps monoclonaux dirigés contre divers états de ladite protéine-test, respectivement dirigés contre ladite protéine-test à l'état natif et dirigés contre des états de dénaturation de ladite protéine-test provoqués par des traitements thermiques effectués à différentes températures, éventuellement voisines les unes des autres, et contre des états de dénaturation de ladite protéine-test provoqués par lesdits traitements thermiques appliqués pendant différentes durées, lesquels anticorps monoclonaux sont aptes à réaliser l'immunocapture de l'antigène correspondant, à savoir de ladite protéine-test dans l'état correspondant aux anticorps dirigés contre elle, ledit kit étant en outre caractérisé en ce qu'il comprend des anticorps polyclonaux propres à permettre l'identification de l'antigène lié à l'anticorps spécifique correspondant.

4° Hybridomes secrétant les anticorps monoclonaux dirigés contre une protéine-test, qui constituent le réactif immunochimique selon l'une quelconque des revendications 1 et 2, déposés auprès de la CNCM tenue par l'INSTITUT PASTEUR en date du 10 Octobre 1989 sous les N° I-907, I-908 et I-909.

FIG.1

FIG.2

FIG.3

FIG.4

EP 0 422 981 A1

FIG.5

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | EP-A-0 343 064 (INSTITUT NATIONAL DE LA RECHER-CHE AGRONOMIQUE)<br>* Document en entier<br>* & FR-A-2 631 705 (Cat. D)<br>— — — | 1-4 | G 01 N<br>33/68<br>G 01 N 33/02<br>C 12 N 5/12 |
| X | CHEMICAL ABSTRACTS, vol. 106, no. 13, 30 mars 1987, page 543, abrégé no. 100990y, Columbus, Ohio, US; P.J. KILSHAW et al.: "Studies on the specificity of antibodies to ovalbumin in normal human serum: technical considerations in the use of ELISA methods",<br>& CLIN. EXP. IMMUNOL. 1986, 66(2), 481-9<br>* Abrégé en entier *<br>— — — | 4 | |
| Y | IDEM<br>— — — | 1-3 | |
| Y | CHEMICAL ABSTRACTS, vol. 105, no. 11, 15 septembre 1986, page 521, colonne 2, abrégé no. 96138m, Columbus, Ohio, US; F.W. JANSSEN et al.: "Detection of soya proteins in heated meat products by "blotting" and "dot blot"",<br>& Z. LEBENSM.-UNTERS. FORSCH. 1986, 182(6), 479-83<br>* Abrégé en entier *<br>— — — | 1-3 | |
| Y | JOURNAL OF FOOD SCIENCE, vol. 53, no. 1, 1988, pages 222-225, Chicago, US; C. BRETON et al.: "Immunochemical properties of native and heat denatured ovalbumin"<br>* Document en entier *<br>— — — | 1-3 | DOMAINES TECHNIQUES RECHERCHES (Int. Cl.5)<br><br>G 01 N |
| Y | JOURNAL OF FOOD SCIENCE, vol. 53, no. 1, 1988, pages 226-230, Chicago, US; C. BRETON et al.: "Immunochemical identification and quantification of ovalbumin additive in canned mushrooms"<br>* Document en entier *<br>— — —<br>—/— | 1-3 | |

Le présent rapport de recherche a été établi pour toutes les revendications

| Lieu de la recherche | Date d'achèvement de la recherche | Examinateur |
|---|---|---|
| La Haye | 07 novembre 90 | GRIFFITH G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

& : membre de la même famille, document correspondant

**Office européen des brevets**

# RAPPORT DE RECHERCHE EUROPEENNE

Numéro de la demande

**EP 90 40 2683**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | JOURNAL OF THE SCIENCE OF FOOD AND AGRICULTURE, vol. 39, 1987, pages 179-184, Oxford, GB; E.K. KANG'ETHE et al.: "Thermostable muscle antigens suitable for use in enzyme immunoassays for identification of meat from various species" * Document en entier * | 1-3 | |
| Y | CHEMICAL ABSTRACTS, vol. 107, no. 5, 3 août 1987, page 558, colonne 2, abrégé no. 38147u, Columbus, Ohio, US; F.W. JANSSEN et al.: "Detection of wheat gluten, whey protein, casein, ovalbumin, and soy protein in heated meat products by electrophoresis, blotting, and immunoperoxidase staining", & J. AGRIC. FOOD. CHEM. 1987, 35(4), 563-7 * Abrégé en entier * | 1-3 | |
| A | AGRICULTURAL AND BIOLOGICAL CHEMISTRY, vol. 49, no. 2, 1985, pages 423-427, Tokyo, JP; Y. KATO et al.: "Alteration of ovalbumin immunogenic activity by glycosylation through maillard reaction" | | |
| A | CHEMICAL ABSTRACTS, vol. 98, no. 9, 28 février 1983, page 501, colonne 2, abrégé no. 70566m, Columbus, Ohio, US; J.W. SISSONS et al.: "Ethanol denaturation of soya bean protein antigens", & J. SCI. FOOD AGRIC. 1982, 33(8), 706-10 | | DOMAINES TECHNIQUES RECHERCHES (Int. Cl.5) |

Le présent rapport de recherche a été établi pour toutes les revendications

| Lieu de la recherche | Date d'achèvement de la recherche | Examinateur |
|---|---|---|
| La Haye | 07 novembre 90 | GRIFFITH G. |

CATEGORIE DES DOCUMENTS CITES
X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons
................................................................
& : membre de la même famille, document correspondant